# EUROPEAN PATENT SPECIFICATION

(11) **EP 4 489 897 B1**
(45) Date of publication and mention of the grant of the patent: **19.08.2026**
(21) Application number: 23718668.9
(22) Date of filing: 07.03.2023
(51) Int. Cl.: B01F 31/441, B01F 33/453, B01F 35/21, B01F 35/50, B01F 35/71, G01N 27/02, G01N 27/06, G01N 33/02, G01N 33/04, G01N 33/14, G01N 35/00, G01N 11/00, G01N 11/12

(54) **MEASURING SYSTEM FOR FOODSTUFFS**
MESSSYSTEM FÜR LEBENSMITTEL
SYSTÈME DE MESURE DESTINÉ AUX PRODUITS ALIMENTAIRES

(30) Priority: 11.03.2022 NL 2031246
(43) Date of publication of application: 15.01.2025
(73) Proprietor: Lanvi Patent B.V., 3147 PB Maassluis (NL)
(72) Inventor: VAN HALSEMA, Frans Emo Diderik, 3147 PB Maassluis (NL)
(74) Representative: Octrooibureau Van der Lely N.V.
(86) International application number: PCT/IB2023/052135
(87) International publication number: WO 2023/170571

(56) References cited:
- WO-A1-2021/066646
- US-A- 6 166 551
- US-A1- 2012 070 813
- US-A1- 2018 008 080
- US-A1- 2019 121 374

## Description

The present invention relates to a measuring system for automated determination and/or monitoring of a quality of a foodstuff mixture, comprising at least one product holder with a longitudinal direction, for receiving a liquid or viscous foodstuff therein, a housing with a receiving space for receiving the at least one product holder, a sensor device for measuring a parameter value of the foodstuff in the product holder, and a control device for controlling the measuring system and the processing of the measured parameter values, further comprising a mixing device, which comprises a mixing body provided in the product holder that comprises a magnetisable or magnetic material, and a displacing device for moving the mixing body in the longitudinal direction, wherein the control device is arranged for driving the displacing device successively so that it imparts a repeated to-and-fro motion to the mixing body.

A measuring system of this kind is known from document WO2021066646-A1, and serves for, among other things, mixing foodstuffs reliably even in narrow product holders.

However, a drawback of the known measuring system is that it is not suitable for many kinds of measurements, and especially not if an additive is to be added to the foodstuff. To obtain a reliable and repeatable measurement of the parameter value it will be necessary for the foodstuff and the added additive to be mixed well. It is then usual that the product holder is opened, the additive is added, the product holder is closed, and the contents are mixed according to a prescribed protocol, which depends on the foodstuff and/or the additive added. The product holder is then put back in the measuring system, after which the new parameter value can be determined.

It will be clear that the required manual operations lead to undesirable errors, and certainly to an unnecessarily large amount of work. In addition, it is not always desirable to open the product holder, for example to prevent substances or microbes etc. getting into the product holder from outside.

It is accordingly an aim of the present invention to provide a measuring system of the stated kind, where the aforementioned drawbacks are no longer present, or at least are reduced.

The invention achieves this aim with a measuring system as claimed in claim 1, in particular a measuring system for automated determination of a quality of a foodstuff mixture, comprising at least one product holder with a longitudinal direction, for receiving a liquid or viscous foodstuff therein, a housing with a receiving space for receiving the at least one product holder, a sensor device for measuring a parameter value of the foodstuff in the product holder, and a control device for controlling the measuring system and the processing of the measured parameter values, further comprising an additive dosing device which is arranged, with the product holder closed, and under the control of the control device, to deliver an additive to the foodstuff in the product holder, and a mixing device, which comprises a mixing body provided in the product holder that comprises a magnetisable or magnetic material, and a displacing device for moving the mixing body in the longitudinal direction, wherein the control device is arranged for driving the displacing device successively so that it imparts a repeated to-and-fro motion to the mixing body in order to mix the foodstuff and the additive to produce the foodstuff mixture. The invention makes use of the insight that if the additive can be delivered automatically in the product holder, and the mixing device is used for mixing, the measurements may be continued without needing to open the product holder. Furthermore, the mixing device can impart well-defined movements to the mixing body. The reliability and the reproducibility of the measurements of the parameter values of the foodstuff mixture will therefore also increase. For there might be total or partial separation, a sediment, and so on. These phenomena could have an adverse effect on the measurements, but may be counteracted by (re)mixing.

In the present invention, the foodstuffs relate to liquid or viscous substances or products, which at least are pumpable. Examples are simply water, but in particular milk or milk products, juices, fruit jellies, oils, sauces, and so on. Because these products are eaten or drunk, it is extremely important to know as much as possible about their properties. Since in addition they are often long-life products, and/or a consumer pack thereof is used for a long time in all kinds of different conditions such as temperature, it is important to know the development in time of these properties. Foodstuffs of this kind are often prepared by adding materials/additives to a base. It is moreover important to know how the additive is incorporated in, or reacts with, the base/the foodstuff. Examples of these are soluble products such as milk powder in water, coffee or tea, drinking chocolate powder in hot or cold milk, soup powder or stock cubes in water, the formation of an emulsion of oil and water, and so on. Moreover, how well a foodstuff resists the action of a substance or a microorganism may be important. Adding them at a known moment, without any chance of unwanted addition of any other substances or microorganisms, provides reliable information about the shelf life of the foodstuff. Sometimes the mixtures of food additives produce phenomena such as separation or the formation of a sediment, which in itself may be undesirable. It is then important to know whether this undesirable change is reversible by mixing. **In** all these and yet other situations, the present invention may be useful, because it not only provides good and reproducible mixing even with viscous foodstuffs, but above all also because the adding of additives does not require any manual operation and the product holder can remain closed.

Particular embodiments are described in the appended subclaims, and will be explained in more detail hereinbelow.

**In** some embodiments, the control device is arranged for carrying out a mixing program by the mixing device, comprising imparting to-and-fro movements to the mixing body at, and/or for, one or more predetermined times, and/or with a predetermined speed and/or amplitude. Although it is possible per se to have the control device receive and process external commands, so as to have the to-and-fro motion carried out by an operator, it is advantageous if this too takes place automatically. Thus, an entire protocol can be entered for carrying out a mixing operation, for example at desired time intervals causing the mixing body to move to and fro a predetermined number of times, or for a defined length of time. Said protocol may for example be dependent on the foodstuff in the product holder, the additive, the parameter to be measured, and so on.

In an important embodiment, the control device is arranged for coordinating said imparting of to-and-fro movements in the mixing program with a series of points in time for carrying out said measurements of the parameter values. In this case the measuring system is arranged to remix the foodstuff with the additive prior to, or on the contrary during, a measurement. Mixing prior to a measurement has the advantage that for example a varying magnetic field (for moving the mixing body) is no longer present, so that electromagnetic measurements are not disturbed thereby.

In some embodiments, the control device is arranged to carry out said mixing program as a function of said delivery of the additive. For example, the foodstuff may be prepared for receiving the additive by carrying out of at least some of the steps of the mixing program. For the foodstuff itself, which may be a complex aggregate of water, proteins, fats and so on, may also be susceptible to separation or the like, which may be counteracted at least partially by mixing beforehand. It will then often be important that this preparing takes place relatively shortly before actual addition of the additive. Moreover, it is also favorable that the measuring system optionally reperforms one or more steps of the mixing program automatically, as a function of addition of the additive. For example, mixing may be carried out starting from shortly before the additive is added, and if required, may continue for some time thereafter. It is also possible that the mixing program comprises several repetitions over time, in other words comprises several mixing operations separated in time, the execution of which advantageously being timed by the control device on the basis of said delivery of additive. For example, in each case the foodstuff/additive mixture may be mixed prior to, or during, a measurement of the parameter value.

The mixing body is movable in the longitudinal direction of the product holder. This provides better mixing than when the mixing body is only rotated about an axis, as in known magnetic agitators. The displacing device can be configured in various ways for movability in the longitudinal direction. For example, the displacing device comprises a stack of at least two magnet coils, controllable separately by the control device, and which are located around the product holder, as described specifically in WO2021066646-A1. The magnetic field generated by the one or more magnet coils can then move the mixing body by attracting it or repelling it. This embodiment not only does not comprise any moving parts, but is also easily adjustable with respect to the strength of the field, and is quick to switch. The displacing device may alternatively also comprise one or more external permanent magnets, which are movable along a guide. For this purpose, the displacing device may comprise a mechanical spindle, or a compressed air or hydraulic device, and so on. An advantage of such embodiments is that they produce no heat, or at least much less heat than coils, said heat possibly having a disturbing effect on the contents of the product holder. By interchanging one or more magnets, it is in addition also possible to adjust the magnetic force for movement.

In some embodiments, the additive dosing device comprises a vessel with a closing means that is to be opened by the control device. The vessel is arranged for receiving the additive, and for delivering the additive under the control of the control device. Delivery is in this way automated.

The closing means serves to keep the vessel, or at least the additive dosing device in general, closed until the control system orders delivery. The closing means is not particularly limited, and may for example be selected as a function of the properties of the additive. In particular, the closing means comprises a valve, such as a one-way valve or duckbill valve. A valve may in this connection also be for example a pivoting part, with locking controllable by the control device, such as a slidable pawl. The additive dosing device may also comprise a piston controllable by the control device, which can force the additive out of the additive dosing device via the valve, such as a one-way valve or duckbill valve. Yet other possibilities are not excluded hereby.

Alternatively or additionally, the closing means comprises for example a detachable cover, which is provided with a first coupling means, wherein the mixing body comprises a second coupling means that is configured to engage on the first coupling means, wherein the control device is arranged to move the mixing body in such a way that the first coupling means and the second coupling means are coupled, and then to move the mixing body away from the additive dosing device, in order to release the additive. Such an embodiment has a great advantage, as the mixing body performs a dual function. On the one hand it mixes the foodstuff and any additive; on the other hand it ensures that the additive can be released from the additive dosing device, and is then in fact a component of the latter. Thus, it is not necessary to provide a complicated and possibly fault-prone construction for delivery. In addition, for example an effective connection between the additive dosing device and the control device, such as a mechanical, electrical or wireless connection, is not in fact necessary, and the effective coupling of the control device with the mixing device that is already present may be sufficient.

The coupling means may be provided in all kinds of forms. In particular, the first coupling means and the second coupling means each comprise a permanent magnet. In this case the first coupling means will be provided in the detachable cover, and the second coupling means in the mixing body. In many embodiments the mixing body will already have a permanent magnet on account of the desired movability under the effect of the displacing device. However, note that it is alternatively possible to configure the second coupling means, in the mixing body, as a magnetisable material, so that coupling with the first coupling means can also be broken easily. In addition, note that a permanent magnet in the detachable cover is in principle also affected by magnetic fields of the mixing device. Often, however, these fields will not need to be very strong, and are in addition relatively uniform. In contrast, the force of attraction, and therefore the final coupling force, between the first and the second coupling means is very strong, depending on the distance between them. Thus, in practice it will be very well possible on the one hand for the strength of the magnet of the first coupling means, and on the other hand for the holding force with which the detachable cover is initially held in place, to be selected to be of a magnitude such that displacement of the mixing body by means of the mixing device does not have the result that the cover is already released thereby. Conversely, the strength of the magnet of the second coupling means may in practice be selected to be great enough so that, after coupling with the first magnet, in combination it will be moved strongly enough by the mixing device to overcome said holding force of the detachable cover, and release the additive.

In other embodiments, the first coupling means and the second coupling means each comprise a complementary component of a detachable or permanent snap coupling. In this case it is sufficient if the mixing body is pressed against the cover by the mixing device and coupling is brought about between the first and the second coupling means. The snap coupling may then for example comprise elastic hooks or the like. Once again, the magnetic strength of the magnetic material in the mixing body should be selected to be large enough to overcome a holding force with which the cover is gripped initially. Of course, a coupling force between the first and the second coupling means should be even greater.

The sensor device in the measuring system according to the invention may take various forms. For example, the sensor device may comprise one or more of a camera, such as for detecting color, turbidity or precipitate, a thermometer, for example of importance because many reactions and quantities are temperature-dependent, and electrodes for measuring electrical quantities such as conductivity or dielectric constant. In some embodiments, the sensor device comprises a viscosimeter for measuring viscosity. Viscosity is an important property of foodstuffs, which is often very time- and/or temperature-dependent, but in addition often changes sharply on addition of an additive. Think for example of the addition of a soup powder to water, or of a binder to a sauce. In an especially attractive embodiment the viscosimeter comprises a series of Hall sensors extending along the product holder for detecting magnetic signals, wherein the control device is arranged for determining the viscosity from the magnetic signals from the Hall sensors. For this purpose, the mixing device causes movement of the mixing body, while the control device receives and processes the signals from the Hall sensors. From the latter it is possible to find the speed of the mixing body, which in its turn is an indication for the viscosity of the contents of the product holder. For further explanation and details of this embodiment, as well as for further particularisation thereof, reference is made expressly to WO2021066646-A1. It is noted here that the embodiment of the present invention now described is particularly attractive because the magnet of the mixing body can perform a triple function. With the magnet, the mixing body can be moved effectively by the foodstuff in the product holder, the viscosity of the foodstuff can be determined, and the cover of a vessel with additive can also be detached, so that the additive can be delivered to the foodstuff, to do further tests with it, without needing to open the product holder, as well as without possibly disturbance-sensitive mechanisms or links with the outside world.

The invention will now be explained in more detail on the basis of some nonlimiting embodiment examples, as well as the drawing. In the drawing:
- Figs. 1A, 1B and 1C show a measuring system 1 according to the invention, in three steps during use, in schematic cross section; and
- Figs. 2A and 2B show schematic views of alternative additive dosing devices.

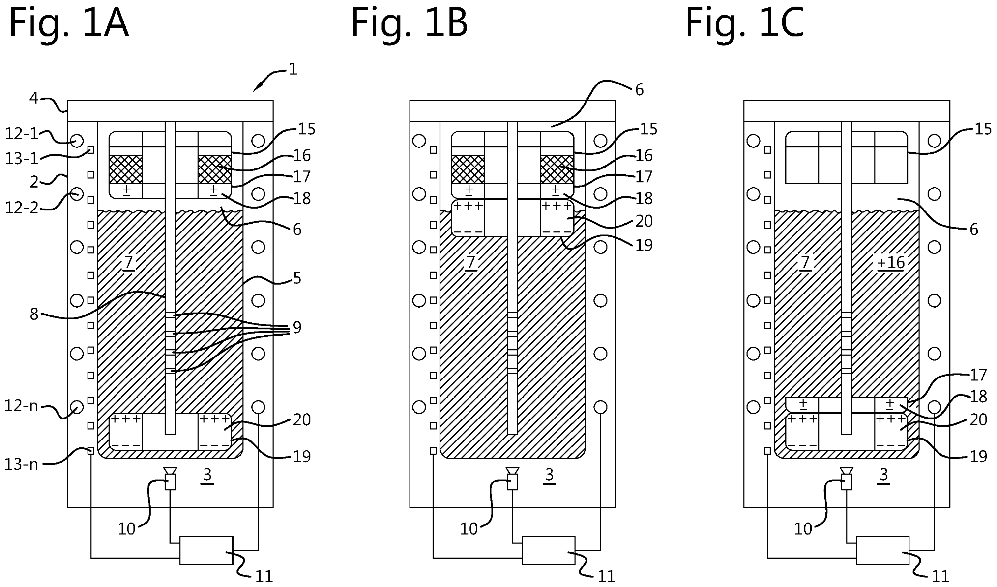

Figs. 1A, 1B and 1C show a measuring system 1 according to the invention, in three steps during use, in schematic cross section, and show in particular a measuring system with a housing 2, a receiving space 3 and a cover 4. A product holder 5 has a product space 6 with in this case a foodstuff 7 therein. A probe is indicated with 8, with four electrodes 9, which are connected, as is a camera 10, to a control device 11.

Magnet coils are indicated with 12-1, 12-2,..., 12-n, and Hall sensors are indicated with 13-1 to 13-n. An additive vessel 15 contains an additive 16, and is closed with a cover 17 with a first magnet 18. A mixer 19 has a second magnet 20.

The measuring system 1 shown here has a housing 2 with a receiving space 3 for only a single product holder 5. It is of course easily possible to provide a larger housing and receiving space, for several product holders. Since said larger number makes no substantial difference for the invention, no further attention will be paid here to that larger number.

The product holder 5 is generally made of glass, for example to allow better observation of the contents, such as with the camera 10, as well as on account of favorable properties of many types of glass, such as chemical resistance. However, other materials, such as stainless steel or the like, are not excluded, wherein the camera will naturally no longer be provided. Here, the product holder 5 is largely filled with a foodstuff 7. The foodstuff is a liquid or viscous foodstuff, such as a dairy product, fruit juice, and so on, but may also relate to water.

The additive vessel 15 contains an additive 16, such as a powder that is to be dissolved in the foodstuff 7, or a substance that is to provoke or simulate a reaction such as ripening or perishing, such as a bacterial or mold culture, and so on. Examples are instant pudding powders, instant coffee or tea, milk powder and so on. The dissolution behavior is important for said additives: how quickly it dissolves, how well, whether lumps are formed, what is the temperature dependence like, and so on. Alternatively, it is important how the foodstuff reacts to the substance or culture added: does spoiling occur, and if so, of what kind and how strongly, and so on.

Measurements on the foodstuff 7, whether or not provided with an additive 16, may be performed for example with a sensor, such as the optional electrodes 9 on the also optional measuring probe 8. These electrodes can measure two at a time, for example the real and imaginary parts of the impedance of the foodstuff, which is a technique known per se. The optional camera 10 would also be able to perform optical measurements, such as relating to the turbidity and/or color of the foodstuff. If required, a light source can be provided (not shown here). Other sensors are also possible. An important example of these is a Hall sensor device, comprising several Hall sensors, indicated here with the reference numbers 13-1 to 13-n, wherein a larger number n, such as between 8 and 20, makes finer detection or monitoring possible. Hall sensors 13 detect a magnetic field, even when it is moving. Thus, the Hall sensors 13 can detect whether and how the mixing body 19 is moving. One or the other is usable in more than one way, for example with the mixing body 19, as will be explained hereunder.

The mixing body 19 may be helpful when dissolving the additive 16. The additive may be added in various ways from the additive vessel 15 to the foodstuff 7. One of these ways is discussed later, when discussing Figs. 1A-C. The mixing body comprises a magnet 20, with which the mixing body is movable up and down by means of the magnet coils 12-1 to 12-n controllable individually by the control device 11, in particular in the manner as already described per se in WO2021066646-A1. In fact the movement from Fig. 1A to Fig. 1B to Fig. 1C is repeated one or more times. Otherwise it is also possible to maintain another amplitude of the movements of the mixing body 19, for example by not energising the upper magnet coil(s). This leads of course to mixing of the added additive 16 in the foodstuff 7.

Mixing should often take place according to a fixed protocol, in order to obtain good, reliable, reproducible measured values. The present invention is very suitable for this, because on the one hand the product holder 5 does not need to be opened, no manual operations are required anyway, and mixing itself takes place completely automatically, and as a result can also take place very reproducibly. Such a protocol may for example stipulate that the mixing body 19 is to be moved to and fro a prescribed number of times over a predetermined distance, and optionally at a predetermined speed. If required, this, or an adapted, mixing operation should be repeated later one or more times. This is easy to carry out with corresponding programming of the control device 11.

Mixing may be coupled by the control device 11 to various situations, even beyond the mixing. Firstly, a mixing operation may be carried out prior to release of the additive 16 from the vessel 15. This may for example serve to homogenise the foodstuff, which is often a complex aggregate of many ingredients, to avoid any negative effects on the subsequent mixing. If required, one or more mixing operations may also be carried out beforehand for better guarantee of the condition of the foodstuff, for example while it ripens or ages. For example, the control device may be arranged to measure one or more parameter values in the foodstuff 7 or mixture 7 + 16 before, during and/or after mixing.

The measuring system according to the invention moreover provides control of the position of the mixing body 19 by means of the second magnet 20 as well as the series of Hall sensors 13-1 to 13-n. As described above, by means of the Hall sensors connected actively thereto, the control device is able to determine the position of the second magnet 20, and therefore of the mixing body 19. By detecting repeatedly, it is also possible to detect the possible movement and the speed of the mixing body. Thus, the control device 11 is able to monitor whether the planned mixing with the mixing body 19 also actually takes place.

Furthermore, by means of the mixing body 19, the separately energisable magnet coils 12 and the Hall sensors 13, the control device 11 is also able to determine viscosity values of the foodstuff. For this purpose it is possible to employ the technique that is described in the aforementioned document WO2021/066646, to which reference is expressly made here. Briefly, the control device 11 moves the mixing body by means of the magnet coils 12 through the foodstuff, and collects the signals that are measured by the Hall sensors 13 during this movement. The control device can determine the speed of movement of the mixing body, in particular from the speed of movement of the peak in the signals. Moreover, since it is known how strongly the magnet coils attract the mixing body 19, and the shape of the mixing body is fixed and is known, the friction on the mixing body can then be determined. The control device 11 can then determine the viscosity of the foodstuff (optionally with the additive) in a manner known per se. It is important to point out that with the present invention a functionality is thus added without increasing the mechanical complexity.

Furthermore, as already stated above, the present invention is usable for opening the additive vessel 15 with its cover 17 with its (first) magnet 18. In Fig. 1A, the mixing body 19 is at the bottom, and the cover 17 closes the additive vessel 15. This closing may for example be achieved because there is a (weak) mechanical connection between cover and vessel, and so on. In this example the magnets 18 and 20 each have an annular shape, as well as a different strength, indicated here with the number of pluses and minuses, the north pole and the south pole respectively. For the purpose of opening the additive vessel 15, the mixing body 19 is moved upward by directed activation of magnet coils 12-1 to 12-n. In Fig. 1B, the mixing body 19 is thus moved upward, and now abuts against the cover 17. The stronger second magnet 20 is now much closer to the first magnet 18 than the first magnet 15, which in addition is weaker. Now, when the magnet coils 12-1 to 12-n are energised directionally again to move the mixing body 19 downward, the mixing body will be able to pull the cover 17 with it, and thus open the additive vessel 15, so that the additive 16 is released and is able to mix with the foodstuff 7. This situation is shown in Fig. 1C.

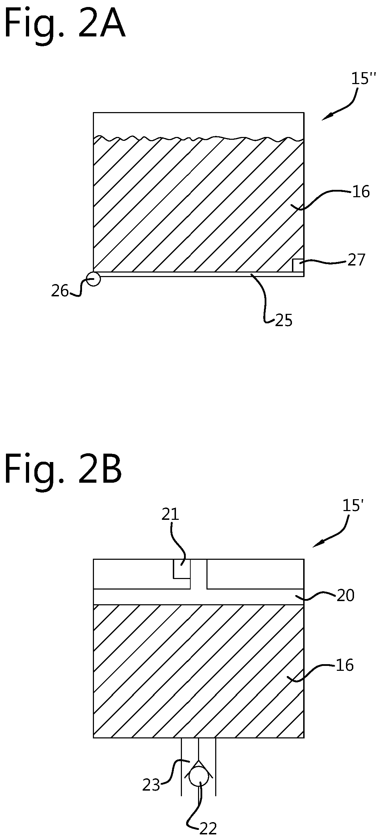

The manner of opening the additive vessel 15 shown in Figs. 1A-C is certainly not the only manner considered. Figs. 2A and 2B show schematic views of some alternative additive dosing devices, indicated with 15' and 15" respectively.

Fig. 2B shows a schematic view of an alternative additive dosing device 15', again with an additive 16, a piston 20 and an actuator 21. A one-way valve is indicated with 22, and an outlet with 23.

This alternative is suitable for example for liquid or otherwise pumpable additives 16, such as an oil or the like. The one-way valve 22 will be selected depending on the properties of the additive. Thus, for a relatively highly viscous oil, a simple nonreturn valve or duckbill will be sufficient to keep the additive 16 in the device 15' until the piston 20 is operated. With a very thin oil or the like, a controllable butterfly valve or the like will preferably be used.

If the control device wants to add additive, it activates the actuator 21 to push the piston 20 away, and thus force the additive 16 via the one-way valve 22 through the outlet 23 to the outside. The additive 16 then reaches the foodstuff (not shown), and can be mixed. This embodiment also does not require any human intervention, but it will be clear that effective coupling must be provided between the actuator 21 and the control device. In addition, owing to the moving parts, this embodiment is more sensitive to disturbances.

Another alternative additive dosing device 15", shown schematically in Fig. 2A, again comprises an additive 16. A cover 25 hinges about pivot 26. A lock is indicated with 27.

In this embodiment, the additive 16 will in principle be released once, when the control system unlocks the lock 27, by means of an unlocking actuator (not shown). The cover 25 will then tilt about the pivot 26, after which gravity causes the additive to be released. It differs from the embodiment in Fig. 2A in that in the latter the additive can be dosed with the piston, and in that the control device may for example be arranged to deliver it in various portions. In contrast, the form in Fig. 2B is simpler mechanically, and more reliable. In addition, this comprises the possibility of using, as unlocking mechanism for the lock 27, a system that coincides with the system in Fig. 1. If the cover 25 is provided with a permanent magnet, preferably near the lock 27, the control device can move, with the magnet coils, the mixing body (not shown), which is also provided with a magnet, against the cover 25. The two magnets, which are now in contact, are then strongly coupled. The control device can then move the combination of mixing body and cover 25 downward, overcoming the resistance of the lock. The cover 25 will tilt about the pivot 26, and also finally come loose from the mixing body again. The additive dosing device 15" is thus able to release the additive 16 without physical connection with the control device, and without actively moving parts.

## Claims

1. A measuring system (1) for automated determination of a quality of a foodstuff mixture (7, 16), comprising
- at least one product holder (5) with a longitudinal direction, for receiving a liquid or viscous foodstuff (7) therein,
- a housing (2) with a receiving space (3) for receiving the at least one product holder,
- a sensor device ((8, 9, 10, 13) for measuring a parameter value of the foodstuff in the product holder, and
- a control device (11) for controlling the measuring system and the processing of the measured parameter values,
further comprising
- an additive dosing device (15, 17; 20; 25), which is arranged, with the product holder closed, and under the control of the control device, to deliver an additive (16) to the foodstuff in the product holder, and
- a mixing device, which comprises:
- a mixing body (19) provided in the product holder that comprises a magnetisable or magnetic material (20), and
- a displacing device (12-1, 12-n) for moving the mixing body in the longitudinal direction,
wherein the control device is arranged for driving the displacing device successively so that it imparts a repeated to-and-fro motion to the mixing body in order to mix the foodstuff and the additive to produce the foodstuff mixture.

2. The measuring system as claimed in claim 1, wherein the control device is arranged for carrying out a mixing program by the mixing device, comprising imparting to-and-fro movements to the mixing body at, and/or for, one or more predetermined times, and/or with a predetermined speed and/or amplitude.

3. The measuring system as claimed in claim 2, wherein the control device is arranged for coordinating said imparting of to-and-fro movements in the mixing program with a series of points in time for carrying out said measurements of the parameter values.

4. The measuring system as claimed in claim 2 or 3, wherein the control device is arranged for carrying out said mixing program as a function of said delivery of the additive.

5. The measuring system as claimed in any one of the preceding claims, wherein the additive dosing device comprises a vessel (15) with a closing means (17; 22; 25) to be opened by the control device.

6. The measuring system as claimed in claim 5, wherein the closing means comprises a valve (22).

7. The measuring system as claimed in claim 5, wherein the closing means comprises a detachable cover (17; 25) that is provided with a first coupling means (18), wherein the mixing body comprises a second coupling means (20) that is configured to engage on the first coupling means, wherein the control device is arranged to move the mixing body in such a way that the first coupling means and the second coupling means are coupled, and then to move the mixing body away from the additive dosing device, in order to release the additive.

8. The measuring system as claimed in claim 7, wherein the first coupling means and the second coupling means each comprise a permanent magnet (18, 20) or a complementary component of a detachable or permanent snap coupling.

## Patentansprüche

1. Messsystem (1) zur automatisierten Bestimmung einer Qualität einer Lebensmittelmischung (7, 16), umfassend
- mindestens einen Produkthalter (5) mit einer Längsrichtung zum Aufnehmen eines flüssigen oder viskosen Lebensmittels (7) darin,
- ein Gehäuse (2) mit einem Aufnahmeraum (3) zur Aufnahme des mindestens einen Produkthalters,
- eine Sensorvorrichtung ((8, 9, 10, 13) zum Messen eines Parameterwertes des Lebensmittels im Produkthalter und
- eine Steuervorrichtung (11) zum Steuern des Messsystems und zum Verarbeiten der gemessenen Parameterwerte;
ferner umfassend
- eine Zusatzstoffdosiervorrichtung (15, 17; 20; 25), die eingerichtet ist, um bei geschlossenem Produkthalter und unter der Steuerung der Steuervorrichtung einen Zusatzstoff (16) an das Lebensmittel im Produkthalter abzugeben, und
- eine Mischvorrichtung, die Folgendes umfasst:
- einen Mischkörper (19), der in dem Produkthalter bereitgestellt ist und ein magnetisierbares oder magnetisches Material (20) umfasst, und
- eine Verschiebevorrichtung (12-1, 12-n) zum Bewegen des Mischkörpers in Längsrichtung,
wobei die Steuervorrichtung eingerichtet ist, um die Verschiebevorrichtung sukzessiv anzutreiben, so dass sie den Mischkörper in eine wiederholte Hin- und Herbewegung versetzt, um das Lebensmittel und den Zusatzstoff zu mischen, um die Lebensmittelmischung herzustellen.

2. Messsystem nach Anspruch 1, wobei die Steuervorrichtung eingerichtet ist, um ein Mischprogramm durch die Mischvorrichtung auszuführen, das umfasst, den Mischkörper zu einer oder mehreren vorbestimmten Zeiten und/oder während eines oder mehrerer Zeiten und/oder mit einer vorbestimmten Geschwindigkeit und/oder Amplitude in Vor- und Rückwärtsbewegungen zu versetzen.

3. Messsystem nach Anspruch 2, wobei die Steuervorrichtung eingerichtet ist, um das Versetzen in Hin- und Herbewegungen im Mischprogramm mit einer Reihe von Zeitpunkten zum Durchführen der Messungen der Parameterwerte zu koordinieren.

4. Messsystem nach Anspruch 2 oder 3, wobei die Steuervorrichtung eingerichtet ist, um das Mischprogramm in Abhängigkeit von der Abgabe des Zusatzstoffes durchzuführen.

5. Messsystem nach einem der vorhergehenden Ansprüche, wobei die Zusatzstoffdosiervorrichtung ein Gefäß (15) mit einem Verschlussmittel (17; 22; 25) umfasst, das von der Steuervorrichtung zu öffnen ist.

6. Messsystem nach Anspruch 5, wobei das Verschlussmittel ein Ventil (22) umfasst.

7. Messsystem nach Anspruch 5, wobei das Verschlussmittel eine abnehmbare Abdeckung (17; 25) umfasst, die mit einem ersten Kopplungsmittel (18) versehen ist, wobei der Mischkörper ein zweites Kopplungsmittel (20) umfasst, das so konfiguriert ist, dass es in das erste Kopplungsmittel eingreift, wobei die Steuervorrichtung eingerichtet ist, um den Mischkörper so zu bewegen, dass das erste Kopplungsmittel und das zweite Kopplungsmittel miteinander gekoppelt werden, und anschließend den Mischkörper von der Zusatzstoffdosiervorrichtung wegzubewegen, um den Zusatzstoff freizugeben.

8. Messsystem nach Anspruch 7, wobei das erste Kopplungsmittel und das zweite Kopplungsmittel jeweils einen Permanentmagnet (18, 20) oder eine komplementäre Komponente einer lösbaren oder permanenten Schnappkopplung umfassen.

## Revendications

1. Système de mesure (1) permettant de déterminer automatiquement une qualité d'un mélange alimentaire (7, 16), comprenant
- au moins un réceptacle à produit (5) ayant une direction longitudinale, destiné à recevoir un aliment liquide ou visqueux (7),
- un boîtier (2) ayant un espace de réception (3) destiné à recevoir l'au moins un réceptacle à produit,
- un dispositif de capteur (8, 9, 10, 13) destiné à mesurer une valeur de paramètre de l'aliment dans le réceptacle à produit, et
- un dispositif de commande (11) destiné à commander le système de mesure et traiter les valeurs de paramètres mesurées,
comprenant en outre
- un dispositif de dosage d'additif (15, 17 ; 20 ; 25), qui est conçu, lorsque le réceptacle à produit est fermé, et sous la commande du dispositif de commande, pour distribuer un additif (16) dans l'aliment dans le réceptacle à produit, et
- un dispositif de mélange qui comprend :
- un corps de mélange (19) disposé dans le réceptacle à produit qui comprend un matériau magnétisable ou magnétique (20), et
- un dispositif de déplacement (12-1, 12-n) destiné à déplacer le corps de mélange dans la direction longitudinale,
dans lequel le dispositif de commande est conçu pour entraîner successivement le dispositif de déplacement de sorte qu'il imprime un mouvement de va-et-vient répété au corps de mélange afin de mélanger l'aliment et l'additif pour produire le mélange alimentaire.

2. Système de mesure selon la revendication 1, dans lequel le dispositif de commande est conçu pour exécuter un programme de mélange par le biais du dispositif de mélange, comprenant l'impression de mouvements de va-et-vient au corps de mélange à un ou plusieurs instants prédéterminés et/ou pendant un ou plusieurs laps de temps prédéterminés, et/ou avec une vitesse et/ou une amplitude prédéterminées.

3. Système de mesure selon la revendication 2, dans lequel le dispositif de commande est conçu pour coordonner ladite impression de mouvements de va-et-vient dans le programme de mélange avec une série de points dans le temps pour exécuter lesdites mesures des valeurs de paramètres.

4. Système de mesure selon la revendication 2 ou 3, dans lequel le dispositif de commande est conçu pour exécuter ledit programme de mélange en fonction de ladite distribution de l'additif.

5. Système de mesure selon l'une quelconque des revendications précédentes, dans lequel le dispositif de dosage d'additif comprend un récipient (15) avec un moyen de fermeture (17 ; 22 ; 25) destiné à être ouvert par le dispositif de commande.

6. Système de mesure selon la revendication 5, dans lequel le moyen de fermeture comprend une vanne (22).

7. Système de mesure selon la revendication 5, dans lequel le moyen de fermeture comprend un couvercle détachable (17 ; 25) qui est pourvu d'un premier moyen d'accouplement (18), dans lequel le corps de mélange comprend un deuxième moyen d'accouplement (20) qui est conçu pour se mettre en prise sur le premier moyen d'accouplement, dans lequel le dispositif de commande est conçu pour déplacer le corps de mélange de façon à ce que le premier moyen d'accouplement et le deuxième moyen d'accouplement s'accouplent, puis pour écarter le corps de mélange du dispositif de dosage d'additif afin de libérer l'additif.

8. Système de mesure selon la revendication 7, dans lequel le premier moyen d'accouplement et le deuxième moyen d'accouplement comprennent chacun un aimant permanent (18, 20) ou un composant complémentaire d'un moyen d'accouplement à emboîtement-pression détachable ou permanent.
